(19) Europäisches Patentamt / European Patent Office / Office européen des brevets

(11) **EP 3 673 979 A1**

(12) **EUROPEAN PATENT APPLICATION**
published in accordance with Art. 153(4) EPC

(43) Date of publication:
**01.07.2020 Bulletin 2020/27**

(51) Int Cl.:
*B01D 61/18* (2006.01)   *C12M 3/00* (2006.01)
*C12M 3/06* (2006.01)

(21) Application number: **18857812.4**

(22) Date of filing: **19.09.2018**

(86) International application number:
**PCT/JP2018/034688**

(87) International publication number:
**WO 2019/059241 (28.03.2019 Gazette 2019/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2017 JP 2017183956**

(71) Applicant: FUJIFILM Corporation
**Tokyo 106-8620 (JP)**

(72) Inventors:
• **TAKAYAMA, Hidetoshi**
Ashigarakami-gun
Kanagawa 258-8577 (JP)
• **YAMASHITA, Hiroshi**
Ashigarakami-gun
Kanagawa 258-8577 (JP)
• **MIYAJIMA, Masumi**
Ashigarakami-gun
Kanagawa 258-8577 (JP)

(74) Representative: **Hoffmann Eitle**
**Patent- und Rechtsanwälte PartmbB**
**Arabellastraße 30**
**81925 München (DE)**

(54) **FILTRATION DEVICE, FILTRATION SYSTEM, AND FILTRATION METHOD**

(57)     Provided is a filtration device including: a flow path that meanders; a filtration membrane that separates a supply side and a permeation side of the flow path; a first distribution port that is provided at one end of the supply side of the flow path; a second distribution port that is provided at the other end of the supply side of the flow path; a first discharge port that is provided on the permeation side of the flow path; and a second discharge port that is provided at a position different from that of the first discharge port on the permeation side of the flow path. First liquid feeding for allowing a liquid flowed in from the first distribution port to flow out from the second distribution port, and second liquid feeding for allowing a liquid flowed in from the second distribution port to flow out from the first distribution port are alternately performed. A liquid that has passed through the filtration membrane is discharged from the first discharge port while the first liquid feeding is performed, and a liquid that has passed through the filtration membrane is discharged from the second discharge port while the second liquid feeding is performed.

FIG. 1

## Description

## BACKGROUND OF THE INVENTION

### 1. Field of the Invention

The disclosed technique relates to a filtration device, a filtration system, and a filtration method.

### 2. Description of the Related Art

The following techniques are known as techniques related to a filtration device including a filtration membrane, for example.

For example, in JP1996-024590A (JP-H08-024590A) discloses in-to-out filtration using a hollow fiber membrane module in which both open ends of a hollow fiber membrane are separately focused and fixed, in which supply of a liquid to be treated to a hollow portion of the hollow fiber membrane is performed by alternately switching from a first liquid inlet and discharge port and a second liquid inlet and discharge port, and immediately after switching a liquid flow direction, filtration is performed while discharging a part of the liquid to be treated from the liquid inlet and discharge port on the supply stop side.

JP2009-291744A discloses a membrane filtration method using a filtration device in which a filtration treatment is performed by a cross flow method in which water to be treated flows from one end side to the other end side of a membrane module along a membrane surface of the membrane module, the filtered water is collected at the other end side.

JP2017-104832A discloses a membrane separation device including a filtration membrane that partitions a casing into a concentrated side space to which water to be treated is supplied and a permeation side space in which permeated water separated from the water to be treated is stored, in which a plurality of U-shaped tubular first connection members that connect one end of a plurality of filtration membranes and the other end so that the plurality of filtration membranes are connected in series.

## SUMMARY OF THE INVENTION

In cell culture, a membrane separation treatment is performed to remove a medium used, dead cells, and the like from a cell suspension. In membrane separation treatment of the cell suspension, a tangential flow method (also referred to as a cross flow method) is often employed in order to minimize damage to cells. The tangential flow method is a method in which a permeation component is sent to a permeation side while flowing a cell suspension that is a target of membrane separation treatment along a membrane surface of the filtration membrane. According to the tangential flow method, it is possible to reduce damage to cells as compared to a dead end method.

In order to realize the mass culture of cells, it is necessary to increase a treatment amount per time of membrane separation treatment, that is, filtration efficiency. As a method for increasing filtration efficiency, increasing a feeding speed of a cell suspension flowing on a membrane surface of a filtration membrane is considered, but in this case, there is a concern about damage to the cells. Accordingly, increasing an area of the filtration membrane is considered effective to as the method for increasing filtration efficiency. For example, an area of a filtration membrane can be increased while maintaining area efficiency by meandering a flow path of the cell suspension flowing on the membrane surface of the filtration membrane. However, in a case where the meandering flow path is formed to increase the area of the filtration membrane, a differential pressure in the membrane surface of the filtration membrane (hereinafter referred to as a filtration pressure) tends to be non-uniform. In a case where a filtration pressure is non-uniform on the membrane surface of the filtration membrane, clogging of cells occurs in a portion where the filtration pressure of the filtration membrane is relatively high, and a cell recovery rate decreases. A decrease in cell recovery rate leads to a decrease in cell culture efficiency, resulting in an increase in the cost of cell culture.

According to one embodiment of the present invention, a filtration device, a filtration system, and a filtration method by which an area of a filtration membrane can be increased while ensuring uniformity in filtration pressure on a membrane surface of the filtration membrane are provided.

A filtration device according to the disclosed technique comprises: a flow path that meanders; a filtration membrane that separates a supply side and a permeation side of the flow path; a first distribution port that is provided at one end of the supply side of the flow path; a second distribution port that is provided at the other end of the supply side of the flow path; a first discharge port that is provided on the permeation side of the flow path; and a second discharge port that is provided at a position different from that of the first discharge port on the permeation side of the flow path.

In a case where a distance from a center of the first distribution port to a farthest position on a membrane surface of the filtration membrane is W1, a distance from the center of the first distribution port to a center of the first discharge port is d1, a distance from a center of the second distribution port to the farthest position on the membrane surface of the filtration membrane is W2, and a distance from the center of the second distribution port to a center of the second discharge port is d2, it is preferable that $0.5W1 \leq d1 \leq W1$ be satisfied, and $0.5W2 \leq d2 \leq W2$ be satisfied, and it is more preferable that $0.7W1 \leq d1 \leq W1$ be satisfied, and $0.7W2 \leq d2 \leq W2$ be satisfied from the viewpoint of inhibiting clogging of cells in the filtration membrane.

The flow path may include a plurality of folded portions, and respective distances from one of the folded portions to a next folded portion may be the same as each other.

One end and the other end of the flow path, and the respective folded portions on one side may be provided on a first straight line, and the respective folded portions on the other side may be provided on a second straight line parallel to the first straight line.

The flow path may include a plurality of folded portions, and a distance from one of the folded portions to a next folded portion may be shorter than other distances.

A filtration system according to the disclosed technique comprises: the above-described filtration device; and a controller that performs first liquid feeding control for controlling liquid feeding of a liquid passing through the first distribution port and the second distribution port, and performs, in conjunction with the first liquid feeding control, second liquid feeding control for controlling liquid feeding of the liquid discharged from the first discharge port and the second discharge port.

The controller may alternately perform, as the first liquid feeding control, first liquid feeding for discharging a liquid flowed in from the first distribution port from the second distribution port, and second liquid feeding for discharging a liquid flowed in from the second distribution port from the first distribution port. The controller may perform, as the second liquid feeding control, discharge of a liquid that has passed through the filtration membrane from the first discharge port while performing the first liquid feeding, and discharge of the liquid that has passed through the filtration membrane from the second discharge port while performing the second liquid feeding. The liquid may be a cell suspension.

A filtration method according to the disclosed technique is a liquid filtration method using the above-described filtration device, the method comprising: alternately performing first liquid feeding for allowing a liquid flowed in from the first distribution port to flow out from the second distribution port, and second liquid feeding for allowing a liquid flowed in from the second distribution port to flow out from the first distribution port; and discharging a liquid that has passed through the filtration membrane from the first discharge port while performing the first liquid feeding, and discharging the liquid that has passed through the filtration membrane from the second discharge port while performing the second liquid feeding. A cell suspension may be a target of the first liquid feeding and the second liquid feeding.

According to one embodiment of the present invention, a filtration device, a filtration system, and a filtration method by which an area of a filtration membrane can be increased while ensuring uniformity in filtration pressure on a membrane surface of the filtration membrane are provided.

## BRIEF DESCRIPTION OF THE DRAWINGS

Fig. 1 is a plan view showing an example of a configuration of a filtration device according to an embodiment of the disclosed technique.
Fig. 2A is a cross-sectional view taken along line 2A-2A in Fig. 1.
Fig. 2B is a cross-sectional view taken along line 2B-2B in Fig. 1.
Fig. 2C is a cross-sectional view taken along line 2C-2C in Fig. 1.
Fig. 2D is a cross-sectional view taken along line 2D-2D in Fig. 1.
Fig. 3 is a plan view showing an example of a configuration of a filtration device according to an embodiment of the disclosed technique.
Fig. 4 is a flowchart showing an example of a filtration method according to an embodiment of the disclosed technique.
Fig. 5A is a view showing an example of distribution of an appropriate filtration pressure region in a case where a filtration method according to an embodiment of the disclosed technique is performed.
Fig. 5B is a graph showing an example of distribution of a filtration pressure at each position on a dashed-dotted line L1 in Fig. 5A.
Fig. 5C is a graph showing an example of distribution of a liquid feeding speed at each position on a dashed-dotted line L1 in Fig. 5A.
Fig. 6A is a view showing an example of distribution of an appropriate filtration pressure region in a case where a filtration method according to an embodiment of the disclosed technique is performed.
Fig. 6B is a graph showing an example of distribution of a filtration pressure on a dashed-dotted line L2 in Fig. 6A.
Fig. 6C is a graph showing an example of distribution of a liquid feeding speed at each position on a dashed-dotted line L2 in Fig. 6A.
Fig. 7A is a view showing overlapped appropriate filtration pressure regions shown in Figs. 5A and 6A.
Fig. 7B is a graph showing overlapped distribution of a filtration pressure shown in Figs. 5B and 6B.
Fig. 7C is a graph showing overlapped distribution of a liquid feeding speed shown in Figs. 5C and 6C.
Fig. 8 is a view showing an example of a configuration of a filtration system according to an embodiment of the disclosed technique.
Fig. 9 is a flowchart showing an example of operation of a controller constituting the filtration system according to an embodiment of the disclosed technique.
Fig. 10 is a view showing an example of distribution of appropriate filtration pressure regions in a case where a filtration method according to First Comparative Example is performed.

Fig. 11 is a view showing an example of distribution of appropriate filtration pressure regions in a case where a filtration method according to Second Comparative Example is performed.

Fig. 12 is a view showing an example of distribution of appropriate filtration pressure regions in a case where a filtration method according to Third Comparative Example is performed.

Fig. 13 is a view showing an example of distribution of appropriate filtration pressure regions in a case where a filtration method according to Fourth Comparative Example is performed.

Fig. 14A is a photograph showing an example of a state of a filtration membrane after performing a membrane separation treatment of a liquid containing simulated particles simulating cells by the filtration method according to Second Comparative Example.

Fig. 14B is a photograph showing an example of a state of a filtration membrane after performing a filtration treatment of a liquid containing simulated particles simulating cells by a filtration method according to an embodiment of the disclosed technique.

Fig. 15 is a plan view showing a configuration of a filtration device according to another embodiment of the disclosed technique.

Fig. 16 is a view showing an example of an appropriate filtration pressure region in a case where a filtration method according to an embodiment of the disclosed technique is performed.

Fig. 17 is a view showing an example of an appropriate filtration pressure region in a case where a filtration method according to another embodiment of the disclosed technique is performed.

Fig. 18 is a plan view showing an example of a configuration of a filtration device according to another embodiment of the disclosed technique.

## DESCRIPTION OF THE PREFERRED EMBODIMENTS

Hereinafter, an example of an embodiment of the disclosed technique will be described with reference to the drawings. In the drawings, the same or equivalent components and parts are denoted by the same reference numerals.

[First embodiment]

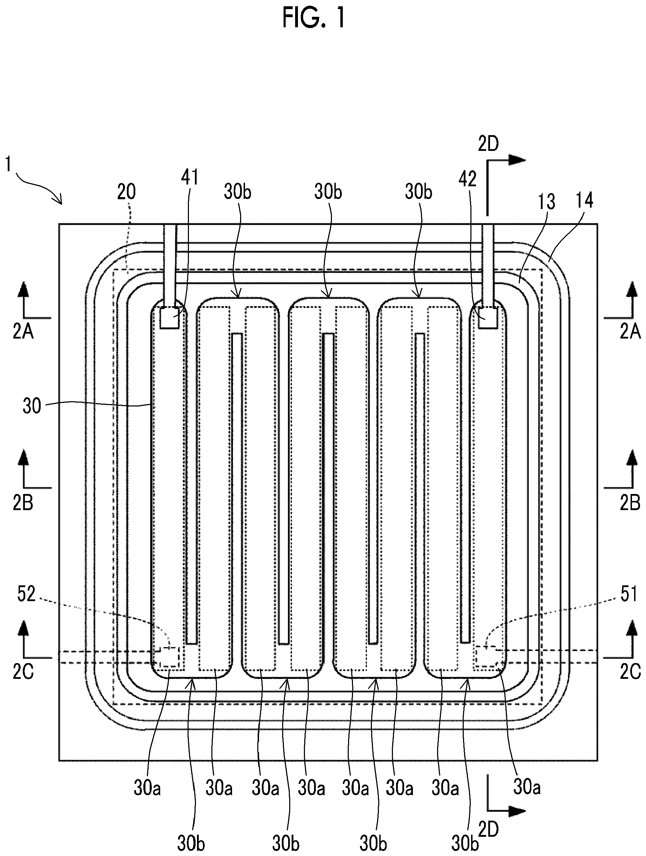

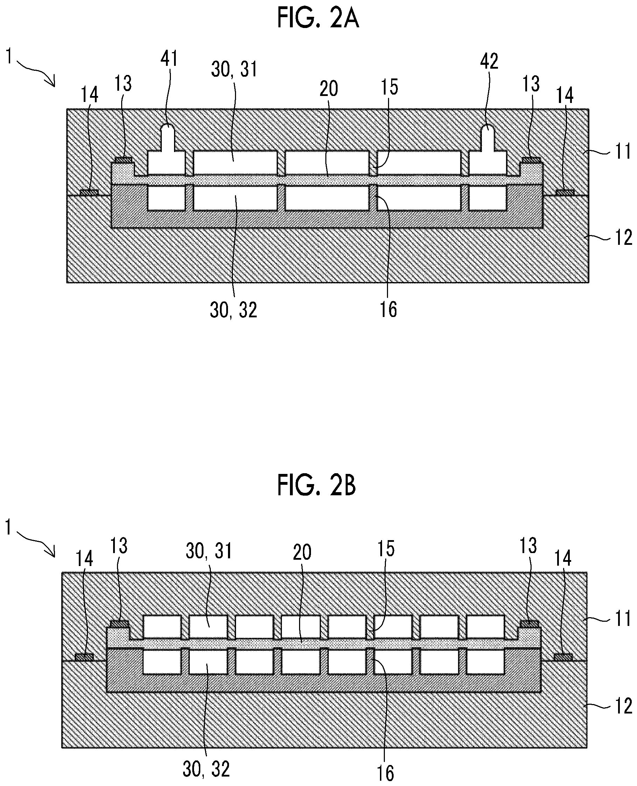

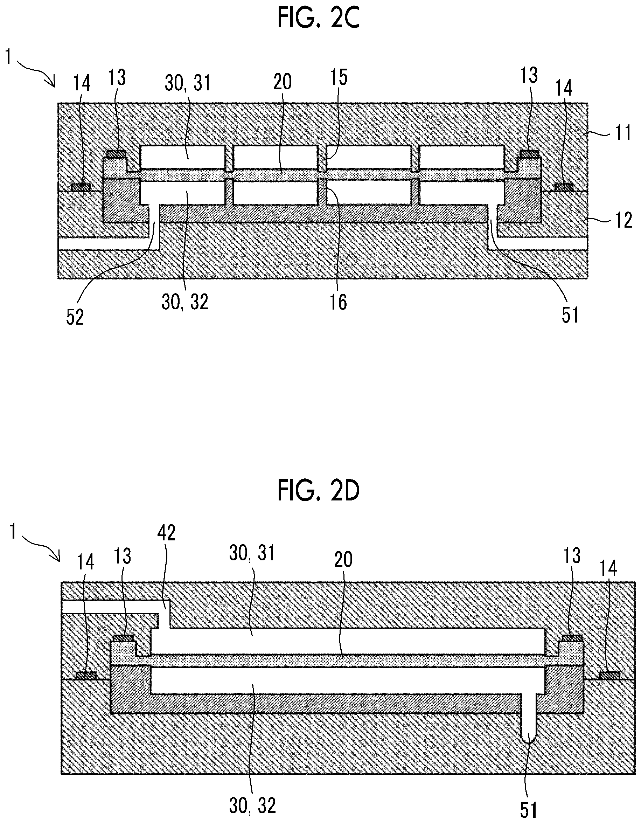

Fig. 1 is a plan view showing an example of a configuration of a filtration device 1 according to an embodiment of the disclosed technique, and Figs. 2A, 2B, 2C, and 2D are respectively a cross-sectional view taken along line 2A-2A, a cross-sectional view taken along line 2B-2B, a cross-sectional view taken along line 2C-2C, and a cross-sectional view taken along line 2D-2D in Fig. 1.

As an example, the filtration device 1 can be used for membrane separation treatment of a cell suspension by a tangential flow method. The filtration device 1 includes an upper case 11 and a lower case 12, and a filtration membrane 20 provided between the upper case 11 and the lower case 12. The filtration device 1 also has an O-ring 13 provided along the outer edge of the filtration membrane 20, and an O-ring 14 surrounding the outer periphery of the filtration membrane 20. The O-ring 13 ensures adhesiveness between the filtration membrane 20 and the upper case 11, and the O-ring 14 ensures adhesiveness between the upper case 11 and the lower case 12.

The upper case 11 and the lower case 12 are respectively provided with projections 15 and 16 that protrude toward the filtration membrane 20 and whose tip portions are in contact with the filtration membrane 20, and a flow path 30 through which a cell suspension passes is formed by these projections 15 and 16. That is, the projections 15 and 16 form a wall surface of the flow path 30. The filtration membrane 20 separates a supply side 31 and a permeation side 32 of the flow path 30. The flow path 30 is configured by connecting a plurality of unit flow paths 30a at a plurality of folded portions 30b. That is, the flow path 30 meanders as shown in Fig. 1.

The filtration membrane 20 has a rectangular shape, and each unit flow path 30a has the same length. That is, each of the one end and the other end of the flow path 30 and the folded portion 30b on one side is provided on a first straight line, and each of the folded portions 30b on the other side is provided on a second straight line parallel to the first straight line. Respective distances from one of the plurality of folded portions 30b to the next folded portion 30b are the same as each other. A case in which respective lengths of the unit flow path 30a are the same as each other means that the difference in length of each of the unit flow paths 30a falls within the range of a processing accuracy error (for example, about 0.05 mm) within the range that can be caused from a cutting process. The filtration membrane 20 has a plurality of pores having a diameter smaller than the diameter of the cells contained in the cell suspension. As the filtration membrane 20, for example, a microfiltration membrane (MF membrane) can be used.

A first distribution port 41 is provided at one end of the supply side 31 of the flow path 30, and a second distribution port 42 is provided at the other end of the supply side 31 of the flow path 30. That is, the first distribution port 41 and the second distribution port 42 are each provided in the vicinity of the corner of the filtration membrane 20 which has a rectangular shape. The cell suspension flows along the membrane surface of the filtration membrane 20 between the first distribution port 41 and the second distribution port 42 on the supply side 31 of the flow path 30. For example, the cell suspension flowing from the first distribution port 41 flows along the supply side 31 of the meandering flow path 30 along the membrane surface of the filtration membrane 20 and flows out from the second distribution port 42. In addition, the cell suspension flowed in from the second distribution port 42 flows along the supply side 31 of the meandering flow path 30 along the membrane surface of the filtration membrane 20 and flows out from the first distribution port 41. The permeation side 32 of the flow path 30 is maintained at a lower pressure than the supply side 31, and while the cell suspension flows through the supply side 31 of the flow path 30, the component smaller than the pore diameter of the filtration membrane 20 is transmitted to the permeation side 32. Cells contained in the cell suspension do not permeate the filtration membrane 20 and flow out of the first distribution port 41 or the second distribution port 42 and are collected.

A first discharge port 51 and a second discharge port 52 for discharging the permeation liquid that has passed through the filtration membrane 20 are provided at different positions on the permeation side 32 of the flow path 30. In the present embodiment, the first discharge port 51 is provided at a position farthest from the first distribution port 41 of the flow path 30, and the second discharge port 52 is provided at a position farthest from the second distribution port 42 in the flow path 30. The "position farthest from the first distribution port 41" refers to a position on the flow path 30 where the straight-line distance from the first distribution port 41 is the longest. In addition, the "position farthest from the second distribution port 42" refers to the position on the flow path 30 where the straight-line distance from the second distribution port 42 is the longest.

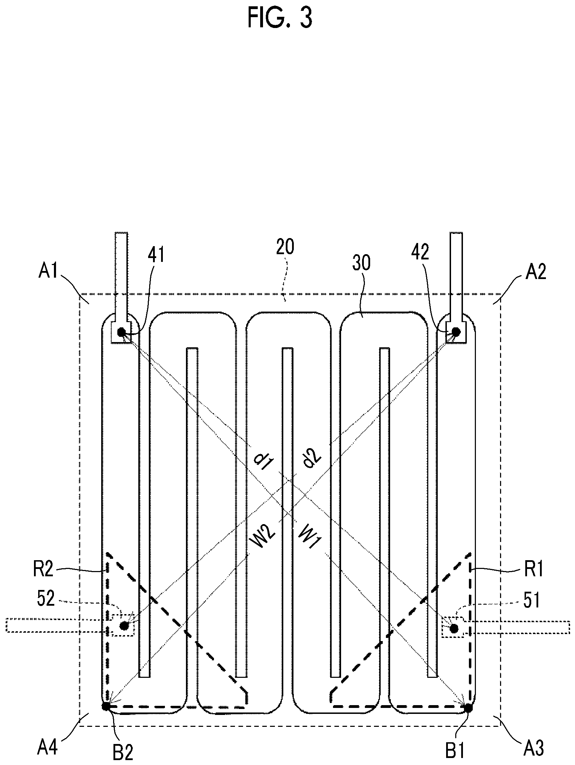

The first discharge port 51 may be provided in the vicinity of the position farthest from the first distribution port 41, and the second discharge port 52 may be provided in the vicinity of the position farthest from the second distribution port 42. That is, as shown in Fig. 3, the first discharge port 51 may be provided in a vicinity region R1 of the corner A3 that is diagonal to the corner A1 of the filtration membrane 20 where the first distribution port 41 is disposed, and the second discharge port 52 may be disposed in a vicinity region R2 of the corner A4 that is diagonal to the corner A2 of the filtration membrane 20 where the second distribution port 42 is disposed.

More specifically, in a case where a distance from a center of the first distribution port 41 to a farthest position B1 on a membrane surface of the filtration membrane 20 is W1, a distance from the center of the first distribution port 41 to a center of the first discharge port 51 is d1, a distance from a center of the second distribution port 42 to the farthest position B2 on the membrane surface of the filtration membrane 20 is W2, and a distance from the center of the second distribution port 42 to a center of the second discharge port 52 is d2, it is preferable that Formula (1) and Formula (2) be satisfied.

$$0.5W1 \leq d1 \leq W1 \qquad (1)$$

$$0.5W2 \leq d2 \leq W2 \qquad (2)$$

From the viewpoint of inhibiting occurrence of cell clogging in the filtration membrane 20, it is more preferable that Formulas (3) and (4) be satisfied.

$$0.7W1 \leq d1 \leq W1 \qquad (3)$$

$$0.7W2 \leq d2 \leq W2 \qquad (4)$$

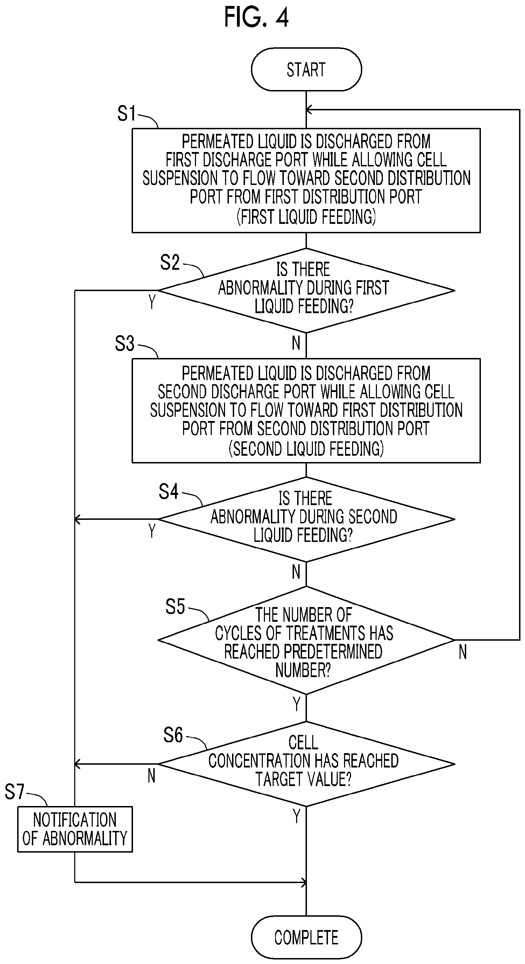

Hereinafter, a filtration method of a cell suspension according to an embodiment of the disclosed technique using the filtration membrane 20 having the above-described configuration will be described with reference to a flowchart shown in Fig. 4.

In Step S1, first liquid feeding is performed in which the cell suspension is caused to flow from the first distribution port 41 and flow out from the second distribution port 42. The cell suspension flowing in from the first distribution port 41 flows along the membrane surface of the filtration membrane 20 while meandering the supply side 31 of the flow path 30. While the cell suspension is flowing through the supply side 31 of the flow path 30, a component smaller than the pore diameter of the filtration membrane 20 contained in the cell suspension permeates the permeation side 32. Thereby, the cell suspension is concentrated. The concentrated cell suspension that has flowed out of the second distribution port 42 is once collected in a container (not shown). In addition, in Step S1, the permeation liquid that has passed through the filtration membrane 20 is discharged from the first discharge port 51 during the first liquid feeding. At this time, the second discharge port 52 is closed so that the permeation liquid is not discharged from the second discharge port 52. In order to promote the discharge of permeation liquid from the first discharge port, a pump may be connected on the flow path communicating with the first discharge port 51, and a suction force may be generated at the first discharge port 51 by operating this pump.

In Step S2, the presence and absence of abnormality during the first liquid feeding is determined. For example, in a case where it is detected that the liquid feeding load in the first liquid feeding is excessive, it may be determined that there is an abnormality as the clogging of the cells in the filtration membrane 20 has occurred. In a case where it is determined that there is an abnormality, the treatment proceeds to Step S7, and it is notified that cell clogging has occurred in the filtration membrane 20. On the other hand, in a case where it is determined that there is no abnormality, the treatment proceeds to Step S3.

Although the rough flow has been described in the flowchart of Fig. 4, Step S2 is repeatedly performed during the first liquid feeding in Step S1. In a case where the condition for ending the first liquid feeding is satisfied while there is no abnormality at the time of the first liquid feeding, the treatment proceeds to Step S3. The condition for ending the first liquid feeding is, for example, the case where all predetermined treatment amounts have been treated. In addition, determination of Step S2 may be performed after the conditions which complete the first liquid feeding are satisfied.

In Step S3, second liquid feeding is performed in which the cell suspension collected in the container is caused to flow from the second distribution port 42 and flow out from the first distribution port 41. That is, a flow direction of the cell suspension flowing through the supply side 31 of the flow path 30 is reversed. The cell suspension flowing in from the second distribution port 42 flows along the membrane surface of the filtration membrane 20 while meandering the supply side of the flow path 30. While the cell suspension is flowing through the supply side 31 of the flow path 30, a component smaller than the pore diameter of the filtration membrane 20 contained in the cell suspension permeates the permeation side 32. Thereby, the cell suspension is concentrated. The concentrated cell suspension that has flowed out of the first distribution port 41 is once collected in a container (not shown). In addition, in Step S3, the permeation liquid that has passed through the filtration membrane 20 is discharged from the second discharge port 52 during the second liquid feeding. At this time, the first discharge port 51 is closed so that the permeation liquid is not discharged from the first discharge port 51. In order to promote the discharge of permeation liquid from the second discharge port, a pump may be connected on the flow path communicating with the second discharge port 52, and a suction force may be generated at the second discharge port 52 by operating this pump.

In Step S4, the presence and absence of abnormality during the second liquid feeding is determined. For example, in a case where it is detected that the liquid feeding load in the second liquid feeding is excessive, it may be determined that there is an abnormality as the clogging of the cells in the filtration membrane 20 has occurred. In a case where it is determined that there is an abnormality, the treatment proceeds to Step S7, and it is notified that cell clogging has occurred in the filtration membrane. On the other hand, in a case where it is determined that there is no abnormality, the treatment proceeds to Step S5.

Although the rough flow has been described in the flowchart of Fig. 4, Step S4 is repeatedly performed during the second liquid feeding in Step S3. In a case where the condition for ending the second liquid feeding is satisfied while there is no abnormality at the time of the second liquid feeding, the treatment proceeds to Step S5. The condition for ending the second liquid feeding is, for example, the case where all predetermined treatment amounts have been treated. In addition, determination of Step S4 may be performed after the conditions which complete the second liquid feeding are satisfied.

In Step S5, it is determined whether or not the number of treatment cycles in which a series of treatment including steps S1 and S2 is one unit has reached a predetermined number. In a case where it is determined that the number of cycles in the series of treatments has not reached the predetermined number, the treatment returns to Step S1. In a case where it is determined that the number of cycles in the series of treatments has reached a predetermined number, the treatment proceeds to Step S6.

In Step S6, it is determined whether or not a concentration of cells in the concentrated cell suspension (hereinafter referred to as cell concentration) has reached a target value. In a case where it is determined that the cell concentration has reached the target value, the treatment ends. On the other hand, in a case where it is determined that the cell concentration has not reached the target value, the treatment proceeds to Step S7 to notify that some abnormality has occurred. In a case where it is determined in Step S6 that the cell concentration has not reached the target value, the treatment may be returned to Step S1.

As described above, in the filtration method of a cell suspension according to the present embodiment, the first liquid feeding that causes the cell suspension flowed in from the first distribution port 41 to flow out from the second distribution port 42, and the second liquid feeding that causes the cell suspension flowed in from the second distribution port 42 to flow out from the first distribution port 41 are alternately performed. Then, the permeation liquid that has permeated through the filtration membrane 20 is discharged from the first discharge port 51 during the first liquid feeding, and the permeation liquid that has permeated through the filtration membrane 20 is discharged from the second discharge port 52 during the second liquid feeding.

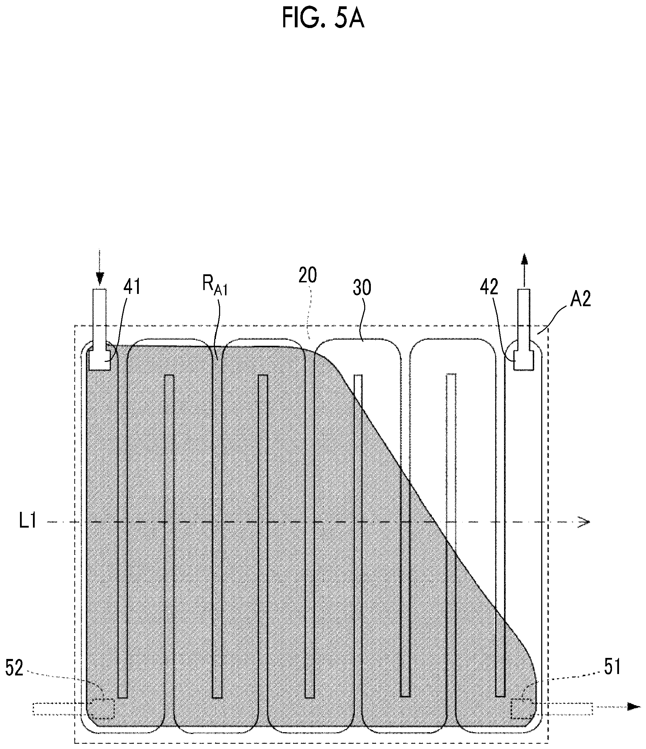

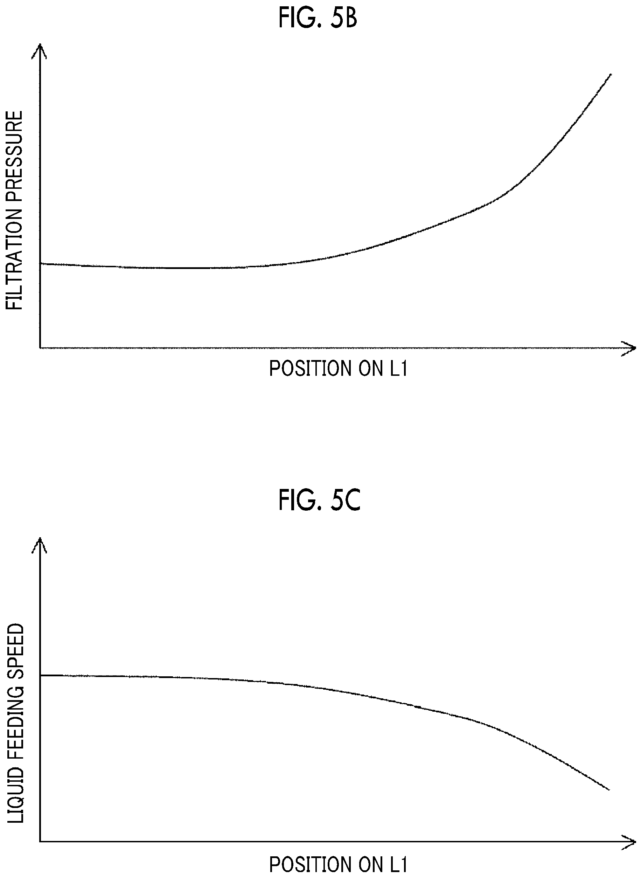

Fig. 5A is a view showing an example of distribution of the region (hereinafter referred to as an appropriate filtration pressure region) $R_{A1}$ in which an appropriate filtration pressure (a membrane surface differential pressure of the filtration membrane 20) in the membrane surface of the filtration membrane 20 has arisen in a case of performing a process of discharging the permeation liquid that has passed through the filtration membrane 20 from the first discharge port 51 (hereinafter referred to as a first process) while performing the first liquid feeding for discharging the cell suspension flowed in from the first distribution port 41 through the second distribution port 42. Fig. 5B is a graph showing an example of distribution of a filtration pressure at each position on a dashed-dotted line L1 in Fig. 5A. Fig. 5C is a graph showing an example of distribution of a liquid feeding speed at each position on a dashed-dotted line L1 in Fig. 5A. A liquid feeding speed refers to the flow rate of the cell suspension that flows on the supply side 31 of the flow path 30.

In the case where the first treatment is performed, the liquid feeding speed on the dashed-dotted line L1 becomes lower as approaching the disposed position of the first discharge port 51 as shown in Fig. 5C. On the other hand, as shown in Fig. 5B, the filtration pressure on the dashed-dotted line L1 increases as the disposed position of the first discharge port 51 is approached. As a result, in the peripheral region of the corner portion A2 where the second distribution port 42 of the filtration membrane 20 is disposed, a region in which the filtration pressure becomes higher than that in the appropriate range (a region not hatched in Fig. 5A, hereinafter referred to as an excessive filtration pressure region) is generated. The excessive filtration pressure region is thought to be related to the generation of a region in which the flow direction of the cell suspension flowing on the supply side 31 of the flow path 30 and the flow direction of the permeation liquid flowing on the permeation side 32 of the flow path 30 are opposite to each other. In the excessive filtration pressure region, clogging of cells is likely to occur, and therefore, it is preferable to inhibit occurrence of the excessive filtration pressure region. In the filtration method according to the present embodiment, while performing the first liquid feeding for causing the cell suspension flowed in from the first distribution port 41 to flow out from the second distribution port 42, the permeation liquid that has permeated through the filtration membrane 20 is discharged from the first discharge port 51 provided at a position farthest from the first distribution port 41 or in the vicinity thereof. This can minimize the area of the region in which the flow direction of the cell suspension flowing on the supply side 31 of the flow path 30 and the flow direction of the permeation liquid flowing on the permeation side 32 of the flow path 30 are opposite to each other. As a result, on the membrane surface of the filtration membrane 20, the area of the appropriate filtration pressure region $R_{A1}$ can be maximized, and the area of the excessive filtration pressure region can be minimized.

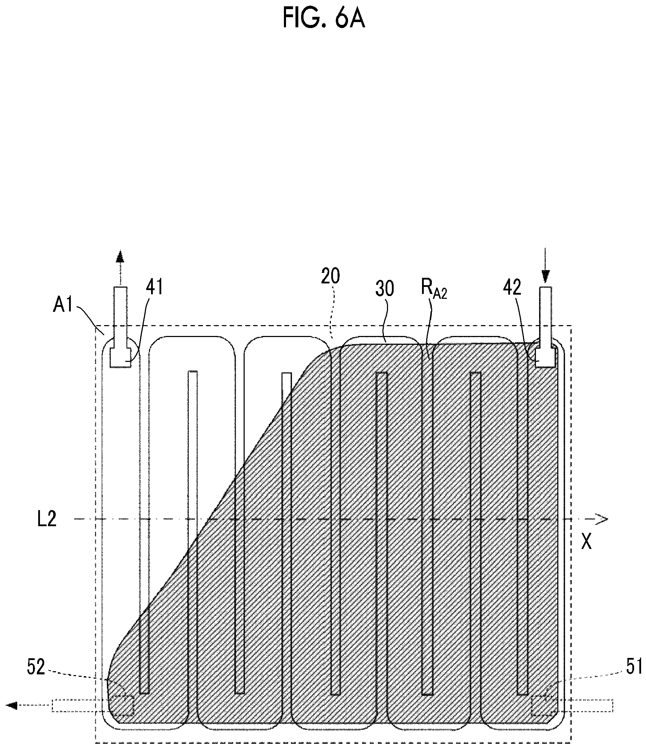

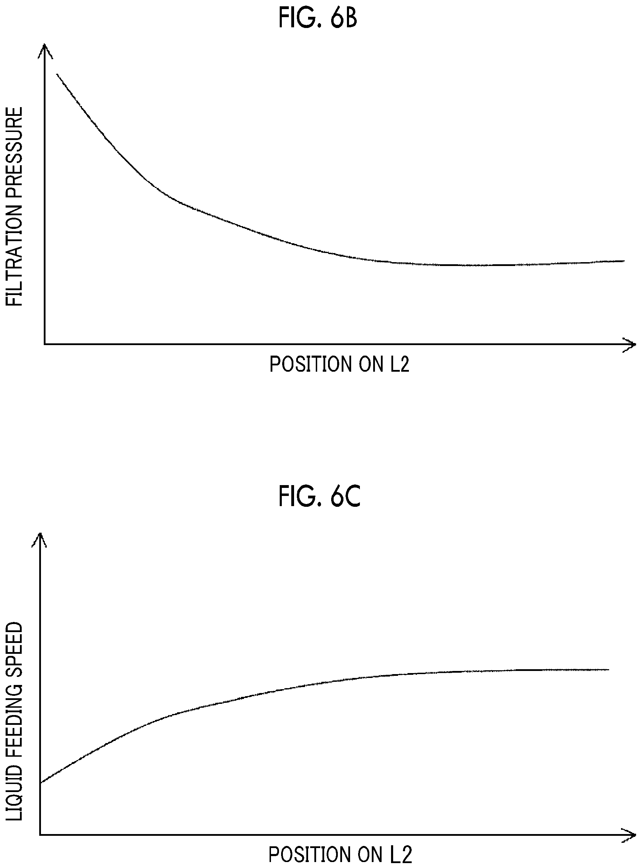

Fig. 6A is a view showing an example of distribution of the appropriate filtration pressure region $R_{A2}$ in the membrane surface of the filtration membrane 20 in a case of performing a process of discharging the permeation liquid that has passed through the filtration membrane 20 from the second discharge port 52 (hereinafter referred to as a second process) while performing the second liquid feeding that causes the cell suspension flowed in from the second distribution port 42 to flow out from the first distribution port 41. Fig. 6B is a graph showing an example of distribution of a filtration pressure on a dashed-dotted line L2 in Fig. 6A. Fig. 6C is a graph showing an example of distribution of a liquid feeding speed at each position on a dashed-dotted line L2 in Fig. 6A.

In the case where the second treatment is performed, the liquid feeding speed on the dashed-dotted line L2 becomes lower as approaching the disposed position of the second discharge port 51 as shown in Fig. 6C. On the other hand, as shown in Fig. 6B, the filtration pressure on the dashed-dotted line L2 increases as the disposed position of the second discharge port 52 is approached. As a result, an excessive filtration pressure region (region not hatched in Fig. 6A) is generated in the peripheral region of the filtration membrane 20 around the corner A1 where the first distribution port 41 is disposed. In the filtration method according to the present embodiment, while performing the second liquid feeding for causing the cell suspension flowed in from the second distribution port 42 to flow out from the first distribution port 41, the permeation liquid that has permeated through the filtration membrane 20 is discharged from the second discharge port 52 provided at a position farthest from the second distribution port 42 or in the vicinity thereof. This can minimize the area of the region in which the flow direction of the cell suspension flowing on the supply side 31 of the flow path 30 and the flow direction of the permeation liquid flowing on the permeation side 32 of the flow path 30 are opposite to each other. As a result, on the membrane surface of the filtration membrane 20, the area of the appropriate filtration pressure region $R_{A2}$ can be maximized, and the area of the excessive filtration pressure region can be minimized.

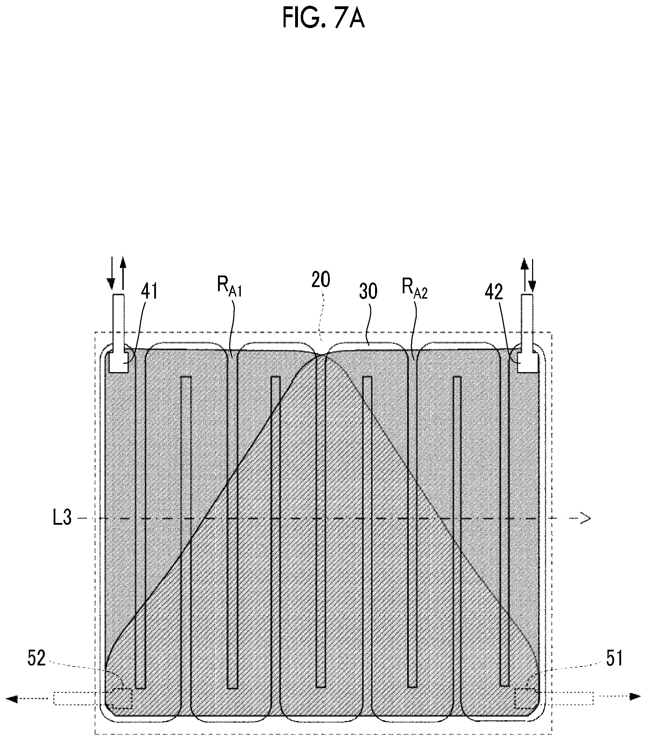

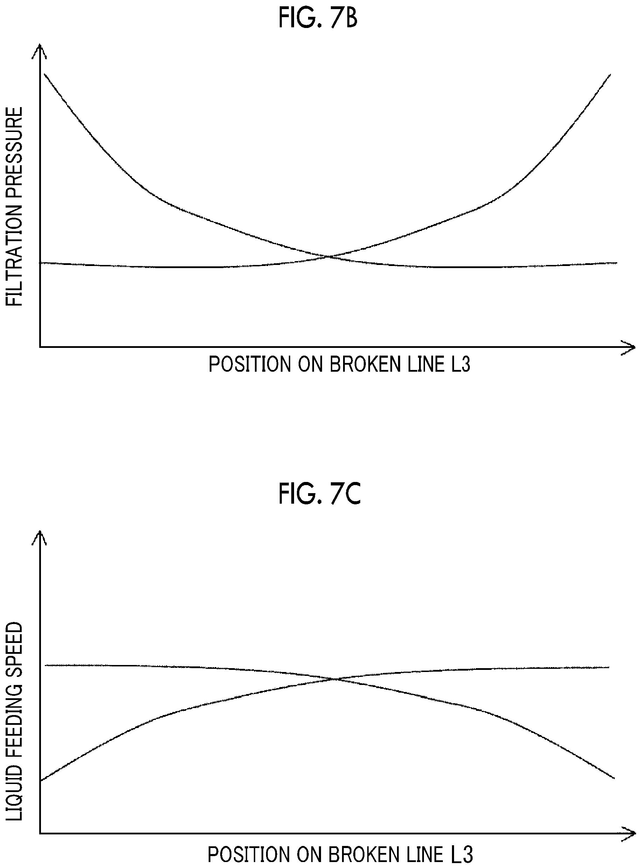

Fig. 7A is a view showing overlapped appropriate filtration pressure regions $R_{A1}$ and $R_{A2}$ shown in Figs. 5A and 6A. Fig. 7B is a graph of overlapped filtration pressure distributions shown in Fig. 5B and Fig. 6B, and is a graph showing an example of the filtration pressure distribution on the dashed-dotted line L3 in Fig. 7A. Fig. 7C is a graph of overlapped liquid feeding speed distributions shown in Fig. 5C and Fig. 6C, and is a graph showing an example of the liquid feeding speed distribution on the dashed-dotted line L3 in Fig. 7A.

In the filtration method according to the present embodiment, the first treatment for discharging the permeation liquid from the first discharge port 51 while performing the first liquid feeding, and the second treatment for discharging the permeation liquid from the second discharge port 52 while performing the second liquid feeding are alternately performed. As a result, the entire excessive filtration pressure region in the first treatment is included in the appropriate filtration pressure region $R_{A2}$ in the second treatment, and the entire excessive filtration pressure region in the second treatment is included in the appropriate filtration pressure region $R_{A1}$ in the first treatment. That is, a region where the appropriate filtration pressure region $R_{A1}$ in the first treatment and the appropriate filtration pressure region $R_{A2}$ in the second treatment are combined covers the entire filtration membrane 20. In addition, the uniformity in time average value of the filtration pressure on the membrane surface of the filtration membrane 20 can be improved. Thereby, it becomes possible to suppress the occurrence of clogging of cells in the filtration membrane 20.

In a case where the length of the filtration membrane 20 in the direction in which the unit flow paths 30a are arranged becomes excessive (that is, in a case where the number of connections of the unit flow paths 30a increases), the entire excessive filtration pressure region in the first treatment is difficult to be included in the appropriate filtration pressure region $R_{A2}$ in the second treatment, and the entire excessive filtration pressure region in the second treatment is difficult to be included in the appropriate filtration pressure region $R_{A1}$ in the first treatment. Accordingly, it is preferable to determine the length of the filtration membrane 20 in the direction in which the unit flow paths 30a are arranged (the number of connected unit flow paths 30a) such that the unit flow path 30a disposed near the center of the filtration membrane 20 in the direction in which the unit flow paths 30a are arranged are included in both the appropriate filtration pressure region $R_{A1}$ in the first process and the appropriate filtration pressure region $R_{A2}$ in the second process.

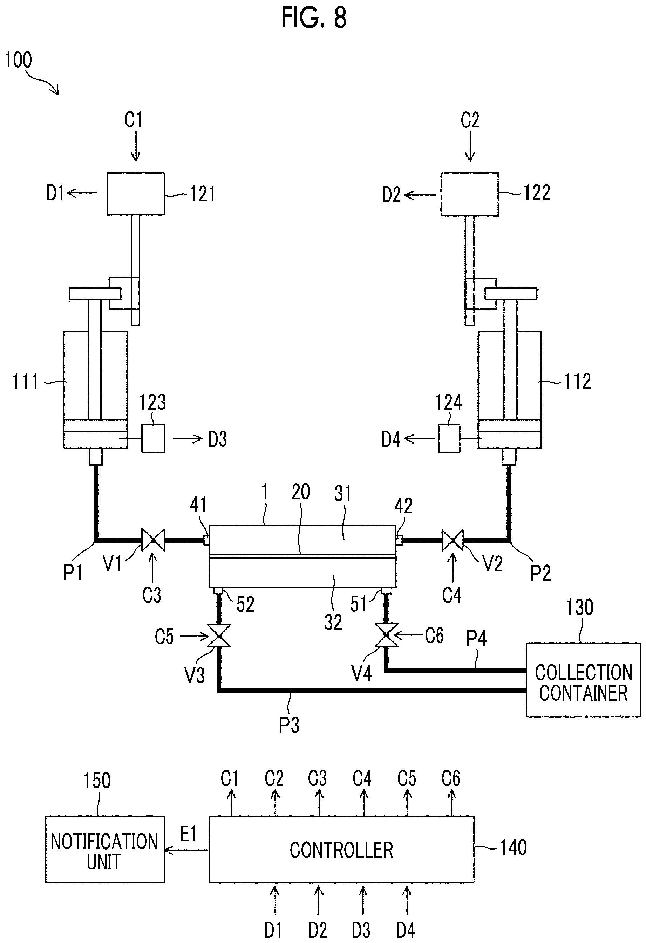

Fig. 8 is a view showing an example of a configuration of a filtration system 100 according to an embodiment of the disclosed technique that realizes the filtration method according to the embodiment of the disclosed technique.

The filtration system 100 includes a filtration device 1, a first syringe 111, a second syringe 112, a first syringe pump 121, a second syringe pump 122, a first cell concentration detection unit 123, and a second cell concentration detection unit 124, a collection container 130, a controller 140, a notification unit 150, valves V1, V2, V3, and V4, and pipes P1, P2, P3, and P4.

Each of the first syringe 111 and the second syringe 112 functions as a container that temporarily stores a cell suspension that is a target of concentration treatment by the filtration device 1, and functions as a part of a cell suspension feeding unit. The jetting port of the first syringe 111 is connected to the first distribution port 41 of the filtration device 1 via the pipe P1. The jetting port of the second syringe 112 is connected to the second distribution port 42 of the filtration device 1 via the pipe P2.

The first cell concentration detection unit 123 outputs a concentration detection signal D3 indicating the concentration of cells accommodated in the first syringe 111. The second cell concentration detection unit 124 outputs a concentration detection signal D4 indicating the concentration of cells accommodated in the second syringe 112.

The first syringe pump 121 has a slide mechanism that slides the plunger of the first syringe 111, and by sliding the plunger of the first syringe 111 according to the control signal C1 supplied from the controller 140, the cell suspension accommodated in the first syringe 111 is fed. The first syringe pump 121 outputs a load amount detection signal D1 indicating the magnitude of the load in a case where the plunger of the first syringe 111 is slid. The load in a case where the plunger of the first syringe 111 is slid increases, for example, as clogging of cells occurs in the filtration membrane 20 and the liquid feeding load increases. Similarly, the second syringe pump 122 has a slide mechanism that slides the plunger of the second syringe 112, and by sliding the plunger of the second syringe 112 according to the control signal C2 supplied from the controller 140, the cell suspension accommodated in the second syringe 112 is fed. The second syringe pump 122 outputs a load amount detection signal D2 indicating the magnitude of the load in a case where the plunger of the second syringe 112 is slid. The load in a case where the plunger of the second syringe 112 is slid increases, for example, as clogging of cells occurs in the filtration membrane 20 and the liquid feeding load increases.

The collection container 130 is a container in which the permeation liquid that has passed through the filtration membrane 20 is collected. The first discharge port 51 of the filtration device 1 is connected to the collection container 130 via the pipe P4, and the second discharge port 52 of the filtration device 1 is connected to the collection container 130 via the pipe P3.

The valve V1 is provided in the middle of the pipe P1, and the valve V2 is provided in the middle of the pipe P2. The valve V3 is provided in the middle of the pipe P3, and the valve V4 is provided in the middle of the pipe P4. Each of the valves V1, V2, V3, and V4 has a form of a solenoid valve, and is controlled to be in an open state or a closed state according to control signals C3, C4, C5, and C6 supplied from the controller 140.

The controller 140 includes, for example, a microcomputer, and performs liquid feeding control in the filtration system 100 by outputting control signals C1 to C6 at a predetermined timing. In addition, the controller 140 determines the presence or absence of abnormality in filtration treatment based on the load amount detection signals D1 and D2 and the concentration detection signals D3 and D4, and outputs an error signal E1 in a case where it is determined that there is an abnormality.

The notification unit 150 notifies that an abnormality has occurred in the filtration system 100 in a case where the error signal E1 is output from the controller 140.

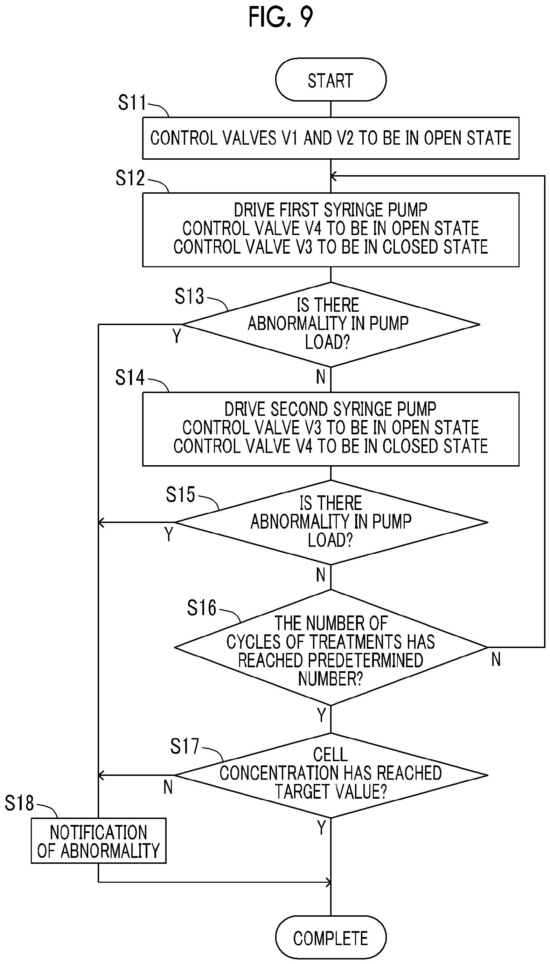

Hereinafter, an example of the operation of the controller 140 will be described with reference to the flowchart shown in Fig. 9. It is assumed that the cell suspension is accommodated in the first syringe 111 as an initial state.

In Step S11, the controller 140 controls the valves V1 and V2 to be in an open state by outputting control signals C3 and C4, respectively.

In Step S12, the controller 140 drives the first syringe pump 121 by outputting the control signal C1. In addition, the controller 140 outputs the control signals C5 and C6, thereby controlling the valve V4 to an open state and controlling the valve V3 to a closed state. Thereby, the cell suspension accommodated in the first syringe 111 flows in into the inside of the filtration device 1 from the first distribution port 41, and the cell suspension flows along the membrane surface of the filtration membrane 20 with the supply side 31 of a flow path to flow out from the second distribution port 42. The cell suspension that has flowed out of the second distribution port 42 is accommodated in the second syringe 112. The permeation liquid that has passed through the filtration membrane 20 is discharged from the first discharge port 51 and stored in the collection container 130.

In Step S13, the controller 140 determines whether an abnormality has occurred in the load amount of the first syringe pump 121 based on the load amount detection signal D1. The controller 140 determines that there is an abnormality when the load amount indicated by the load amount detection signal D1 exceeds a predetermined amount, and otherwise determines that there is no abnormality. In a case where the controller 140 determines that the load amount of the first syringe pump 121 is abnormal, the treatment proceeds to Step S18. In Step S18, the controller 140 outputs an error signal E1. Based on the error signal E1, the notification unit 150 notifies that the cell clogging has occurred in the filtration membrane 20.

In Step S14, the controller 140 drives the second syringe pump 122 by outputting the control signal C2. In addition, the controller 140 outputs the control signals C5 and C6, thereby controlling the valve V3 to an open state and controlling the valve V4 to a closed state. Thereby, the cell suspension accommodated in the second syringe 112 flows in into the inside of the filtration device 1 from the second distribution port 42, and the cell suspension flows along the membrane surface of the filtration membrane 20 with the supply side 31 of a flow path to flow out from the first distribution port 41. The cell suspension that has flowed out of the first distribution port 41 is accommodated in the first syringe 111. The permeation liquid that has passed through the filtration membrane 20 is discharged from the second discharge port 52 and stored in the collection container 130.

In Step S15, the controller 140 determines whether an abnormality has occurred in the load amount of the second syringe pump 122 based on the load amount detection signal D2. The controller 140 determines that there is an abnormality when the load amount indicated by the load amount detection signal D2 exceeds a predetermined amount, and otherwise determines that there is no abnormality. In a case where the controller 140 determines that the load amount of the second syringe pump 122 is abnormal, the treatment proceeds to Step S18. In Step S18, the controller 140 outputs an error signal E1. Based on the error signal E1, the notification unit 150 notifies that the cell clogging has occurred in the filtration membrane 20.

In Step S16, the controller 140 determines whether or not the number of treatment cycles in which a series of treatment including each treatment from step S12 to step S15 is one unit has reached a predetermined number. In a case where the controller 140 determines that the number of cycles of the series of treatments has not reached the predetermined number, the treatment returns to Step S12. On the other hand, in a case where the controller 140 determines that the number of cycles of the series of treatments has reached a predetermined number, the treatment proceeds to Step S17.

In Step S17, the controller 140 determines whether the concentration of cells that have undergone each of the treatments has reached a target value, which is indicated by the concentration detection signal D1 output from the first cell concentration detection unit 123 or the concentration detection signal D2 output from the second cell concentration detection unit 124. In a case where the controller 140 determines that the concentration of cells that have undergone each of the treatments has not reached the target value, the treatment proceeds to Step S18. The notification unit 150 notifies that some abnormality has occurred in the filtration system 100 based on the error signal E1. On the other hand, in a case where it is determined that the concentration of cells that have undergone each treatment has reached the target value, the controller 140 ends the treatment. In addition, the controller 140 resets a count value of a treatment cycle number in a case where it determines that the concentration of cells that have undergone each treatment has not reached the target value, and each treatment from Step S11 to Step S16 may be performed again.

As described above, the controller 140 controls the first syringe pump 121 and the second syringe pump 122 to perform the first liquid feeding control that controls liquid feeding of a liquid passing through the first distribution port 41 and the second distribution port 42. In addition, the controller 140 performs opening and closing control of the valves V3 and V4 in conjunction with the first liquid feeding control, thereby performing the second liquid feeding control for controlling the liquid feeding of the permeation liquid discharged from the first discharge port 51 and the second discharge port 52. Specifically, the controller 140 alternately performs, as the first liquid feeding control, the first liquid feeding for discharging a cell suspension flowed in from the first distribution port 41 from the second distribution port 42, and the second liquid feeding for discharging a cell suspension flowed in from the second distribution port 42 from the first distribution port 41. As the second liquid feeding control, the controller 140 discharges the permeation liquid that has permeated through the filtration membrane 20 from the first discharge port 51 during the first liquid feeding, and discharges the permeation liquid that has permeated through the filtration membrane 20 from the second discharge port 52 during the second liquid feeding.

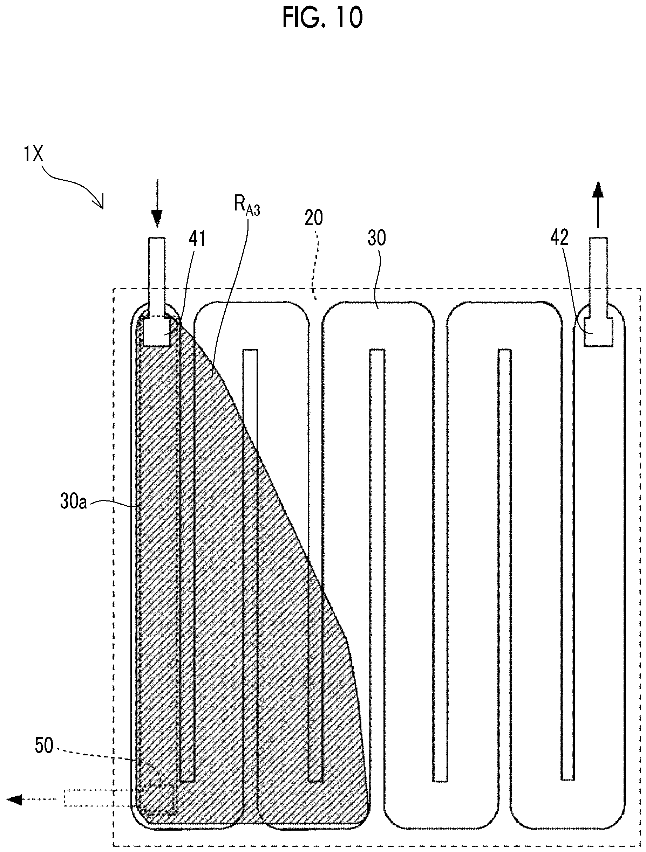

Fig. 10 is a diagram showing an example of the distribution of the appropriate filtration pressure region $R_{A3}$ on the membrane surface of the filtration membrane 20 in a case where the filtration method according to the First Comparative Example using a filtration device IX according to the comparative example is performed.

The filtration device IX has only one discharge port for discharging the permeation liquid that has passed through the filtration membrane 20. In the filtration device IX according to the comparative example, a discharge port 50 is provided on a permeation side, which is opposite to the end on the side where the first distribution port 41 is provided, of an end of the unit flow path 30a to which the first distribution port 41 is connected. In the filtration method according to First Comparative Example, a treatment of discharging the permeation liquid from the discharge port 50 while the cell suspension flowed in from the first distribution port 41 flows out of the second distribution port 42 is continuously performed. That is, the filtration method according to First Comparative Example does not switch the liquid feeding direction on the supply side.

According to the filtration method according to First Comparative Example using the filtration device IX, due to the disposition of the discharge ports 50, the area of the appropriate filtration pressure region $R_{A3}$ is smaller than the area of the appropriate filtration pressure region $R_{A1}$ in a case where the filtration method according to the embodiment of the disclosed technique is applied (refer to Figs. 5A to 5C). The appropriate filtration pressure region $R_{A3}$ does not cover the entire filtration membrane 20. Accordingly, according to the filtration method according to First Comparative Example, as compared with the filtration method according to the embodiment of the disclosed technique, the risk of occurrence of cell clogging increases, and the cell recovery rate decreases.

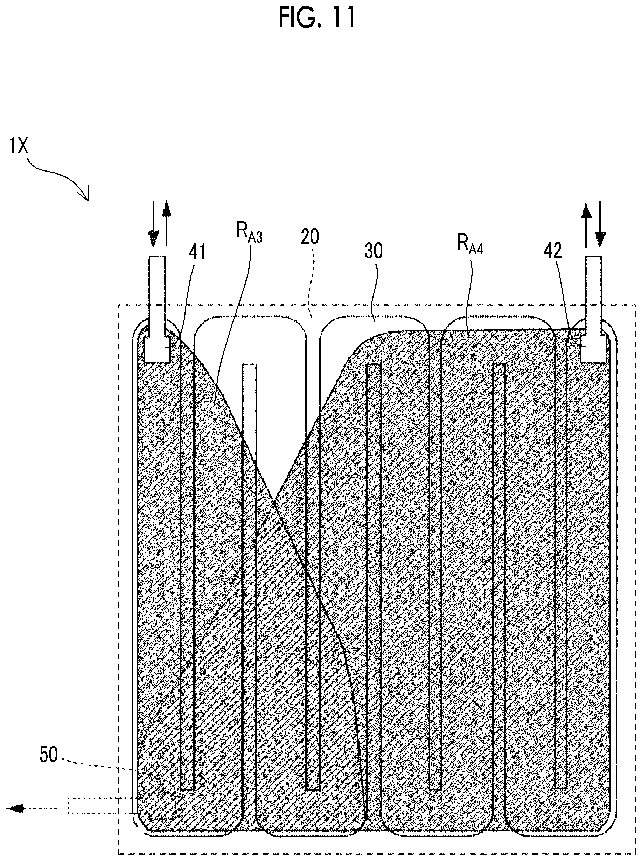

Fig. 11 is a diagram showing an example of the distribution of the appropriate filtration pressure regions $R_{A3}$ and $R_{A4}$ on the membrane surface of the filtration membrane 20 in a case where the filtration method according to Second Comparative Example using a filtration device IX is performed. In the filtration method according to Second Comparative Example, a first treatment for discharging the permeation liquid that has passed through the filtration membrane 20 from the discharge port 50 while performing the first liquid feeding that causes the cell suspension flowed in from the first distribution port 41 to flow out from the second distribution port 42, and a second treatment of discharging the permeation liquid that has passed through the filtration membrane 20 from the discharge port 50 while performing the second liquid feeding that causes the cell suspension flowed in from the second distribution port 42 to flow out from the first distribution port 41 are performed alternately.

According to the filtration method according to Second Comparative Example, a part of the excessive filtration pressure region in the first treatment can be included in the appropriate filtration pressure region $R_{A4}$ in the second treatment, and a part of the excessive filtration pressure region in the second treatment can be included in the appropriate filtration pressure region $R_{A3}$ in the first treatment. However, due to the fact that the number of discharge port for discharging the permeation liquid is one, the entire excessive filtration pressure region in the first treatment cannot be included in the appropriate filtration pressure region $R_{A4}$ in the second treatment. In addition, the entire excessive filtration pressure region in the second treatment cannot be included in the appropriate filtration pressure region $R_{A3}$ in the first treatment. That is, a region where the appropriate filtration pressure region $R_{A3}$ in the first treatment and the appropriate filtration pressure region $R_{A4}$ in the second treatment are combined does not cover the entire filtration membrane 20. Accordingly, according to the filtration method according to Second Comparative Example, as compared with the filtration method according to the embodiment of the disclosed technique, the risk of occurrence of cell clogging increases, and the cell recovery rate decreases.

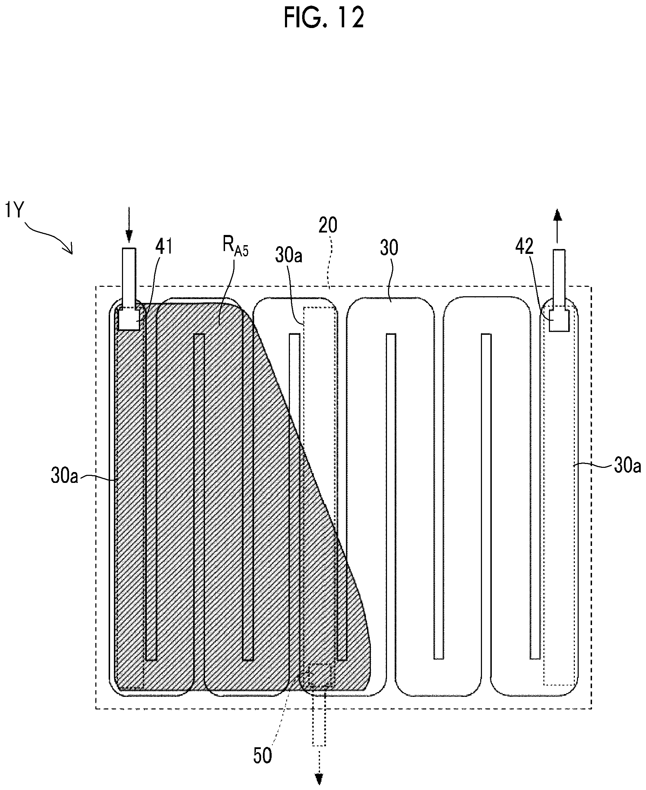

Fig. 12 is a diagram showing an example of the distribution of the appropriate filtration pressure region $R_{A5}$ on the membrane surface of the filtration membrane 20 in a case where the filtration method according to Third Comparative Example using a filtration device 1Y according to the comparative example is performed.

The filtration device 1Y has only one discharge port for discharging the permeation liquid that has passed through the filtration membrane 20. In the filtration device 1Y according to the comparative example, the discharge port 50 is provided on the permeation side of the end, which is opposite to the end on which the first distribution port 41 and the second distribution port 42 are provided, of the unit flow path 30a located between the unit flow path 30a provided with the first distribution port 41 and the unit flow path 30a connected with the second distribution port 42. In the filtration method according to Third Comparative Example, a treatment of discharging the permeation liquid from the discharge port 50 while the cell suspension flowed in from the first distribution port 41 flows out of the second distribution port 42 is continuously performed. That is, the filtration method according to Third Comparative Example does not switch the liquid feeding direction on the supply side.

According to the filtration method according to Third Comparative Example using the filtration device 1Y, the area of the appropriate filtration pressure region $R_{A5}$ becomes larger than the area of the appropriate filtration pressure region $R_{A3}$ in a case where the filtration method according to First Comparative Example using the filtration device IX is performed (refer to Figs. 5A to 5C). However, since the disposition of the discharge ports 50 is not optimal, the area of the appropriate filtration pressure region $R_{A5}$ is smaller than the area of the appropriate filtration pressure region $R_{A1}$ in a case where the filtration method according to the embodiment of the disclosed technique is performed (refer to Figs. 5A to 5C). Furthermore, The appropriate filtration pressure region $R_{A5}$ does not cover the entire filtration membrane 20. Accordingly, according to the filtration method according to Third Comparative Example, as compared with the filtration method according to the embodiment of the disclosed technique, the risk of occurrence of cell clogging increases, and the cell recovery rate decreases.

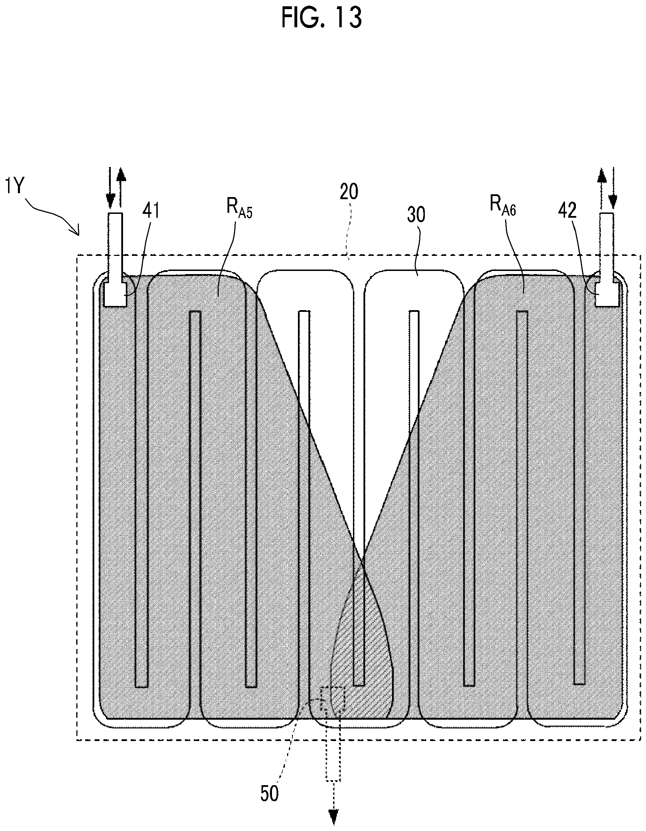

Fig. 13 is a diagram showing an example of the distribution of the appropriate filtration pressure regions $R_{A5}$ and $R_{A6}$ on the membrane surface of the filtration membrane 20 in a case where the filtration method according to Fourth Comparative Example using a filtration device 1Y is performed. In the filtration method according to Fourth Comparative Example, a first treatment for discharging the permeation liquid that has passed through the filtration membrane 20 from the discharge port 50 while performing the first liquid feeding that causes the cell suspension flowed in from the first distribution port 41 to flow out from the second distribution port 42, and a second treatment of discharging the permeation liquid that has passed through the filtration membrane 20 from the discharge port 50 while performing the second liquid feeding that causes the cell suspension flowed in from the second distribution port 42 to flow out from the first distribution port 41 are performed alternately.

According to the filtration method according to Fourth Comparative Example, a part of the excessive filtration pressure region in the first treatment can be included in the appropriate filtration pressure region $R_{A6}$ in the second treatment, and a part of the excessive filtration pressure region in the second treatment can be included in the appropriate filtration pressure region $R_{A5}$ in the first treatment. However, due to the fact that the number of discharge port for discharging the permeation liquid is one, and the disposition thereof is not optimal, the entire excessive filtration pressure region in the first treatment cannot be included in the appropriate filtration pressure region $R_{A6}$ in the second treatment. In addition, the entire excessive filtration pressure region in the second treatment cannot be included in the appropriate filtration pressure region $R_{A5}$ in the first treatment. That is, a region where the appropriate filtration pressure region $R_{A5}$ in the first treatment and the appropriate filtration pressure region $R_{A6}$ in the second treatment are combined does not cover the entire filtration membrane 20. Accordingly, according to the filtration method according to Fourth Comparative Example, as compared with the filtration method according to the embodiment of the disclosed technique, the risk of occurrence of cell clogging increases, and the cell recovery rate decreases.

On the other hand, in the filtration method using the filtration device 1 according to the embodiment of the disclosed technique, a first treatment of discharging the permeation liquid from the first discharge port 51 provided at the farthest position from the first distribution port 41 or in the vicinity thereof while performing the first liquid feeding that causes the cell suspension flowed in from the first distribution port 41 to flow out from the second distribution port 42, and a second treatment of discharging the permeation liquid from the second discharge port 52 provided at the farthest position from the second distribution port 42 or in the vicinity thereof while performing the second liquid feeding that causes the cell suspension flowed in from the second distribution port 42 to flow out from the first distribution port 41 are alternately performed. As a result, the entire excessive filtration pressure region in the first treatment is included in the appropriate filtration pressure region $R_{A2}$ in the second treatment, and the entire excessive filtration pressure region in the second treatment is included in the appropriate filtration pressure region $R_{A1}$ in the first treatment. That is, a region where the appropriate filtration pressure region $R_{A1}$ in the first treatment and the appropriate filtration pressure region $R_{A2}$ in the second treatment are combined covers the entire filtration membrane 20. In addition, a time average value of the filtration pressure on the membrane surface of the filtration membrane 20 can be made substantially uniform. Therefore, the risk of occurrence of cell clogging can be inhibited, and the cell recovery rate can be increased as compared with First to Fourth Comparative Examples.

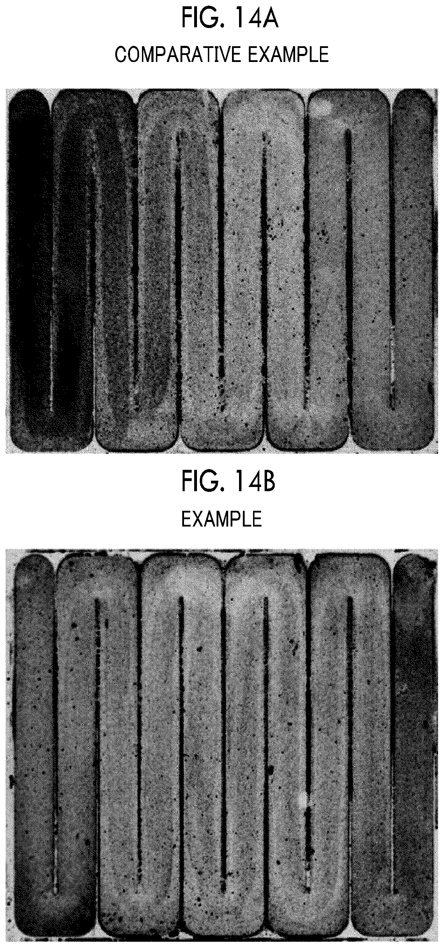

Fig. 14A is a photograph showing a state of a filtration membrane after performing a membrane separation treatment of a liquid containing simulated particles simulating cells by the filtration method according to Second Comparative Example using the filtration device IX (refer to Fig. 11). Fig. 14B is a photograph showing an example of a state of a filtration membrane after performing a membrane separation treatment of a liquid containing simulated particles simulating cells by a filtration method according to an embodiment of the disclosed technique using the filtration device 1. In Figs. 14A and 14B, colored portions are portions where simulated particles are deposited, and portions with a high coloration concentration are portions with a relatively high filtration pressure.

According to the filtration method according to Second Comparative Example, as shown in Fig. 14A, a portion with a high coloration concentration and a portion with a low coloration concentration are clearly separated. This indicates that the difference between the high filtration pressure portion and the low filtration pressure portion is remarkable on the membrane surface of the filtration membrane, that is, the filtration pressure on the membrane surface of the filtration membrane is not uniform. On the other hand, according to the filtration method according to the embodiment of the disclosed technique, the coloration concentration is generally uniform as shown in Fig. 14B. This indicates that the filtration pressure on the membrane surface of the filtration membrane is substantially uniform.

As described above, the filtration device 1, the filtration system 100, and the filtration method according to the embodiment of the disclosed technique, it is possible to increase the area of the filtration membrane while ensuring the uniformity of the filtration pressure on the membrane surface of the filtration membrane 20. Therefore, the filtration efficiency can be increased while suppressing a decrease in the cell recovery rate.

[Second embodiment]

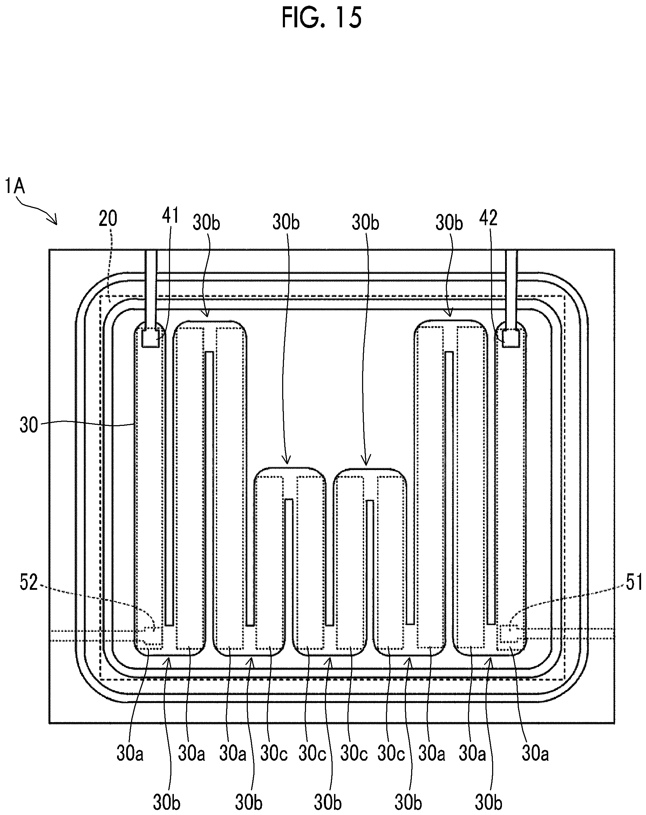

Fig. 15 is a plan view showing a configuration of a filtration device 1A according to a second embodiment of the disclosed technique. The filtration device 1A is different from the filtration device 1 according to the first embodiment in that the flow path 30 has a plurality of unit flow paths 30a, and a plurality of unit flow paths 30c whose flow path length is shorter than that of the unit flow paths 30a. In other words, in the filtration device 1A, the flow path 30 has a portion where the distance from one of the folded portions 30b to the next folded portion 30b is shorter than the other portions. In a case where the length of the unit flow path 30a is La and the length of the unit flow path 30c is Lc, it is preferable that Formula (5) be satisfied.

$$0.5 \times La < Lc < La \qquad (5)$$

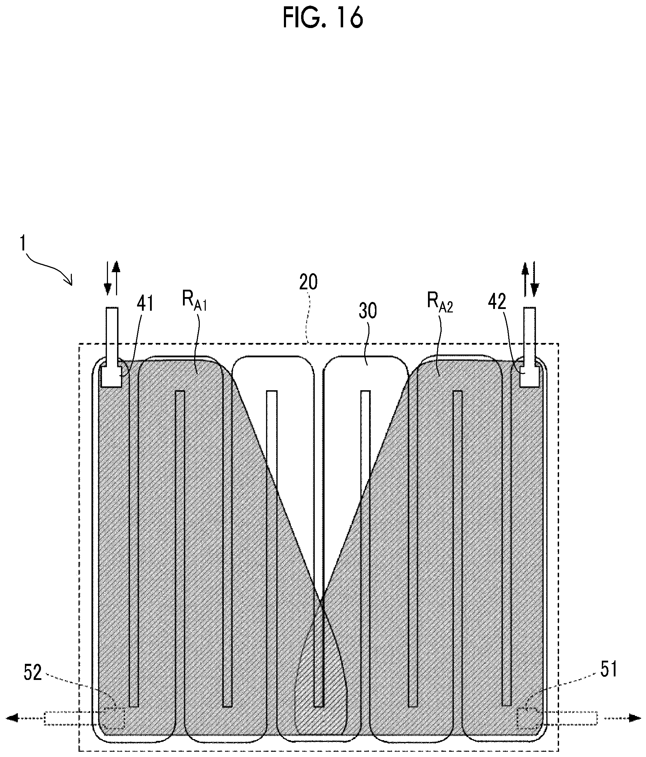

For example, in a case where the first treatment of discharging the permeation liquid from the first discharge port 51 while performing the first liquid feeding for causing the cell suspension flowed in from the first distribution port 41 to flow out from the second distribution port 42, and the second treatment of discharging the permeation liquid from the second discharge port 52 while performing the second liquid feeding for causing the cell suspension flowed in from the second distribution port 42 to flow out from the first distribution port 41 are alternately performed using the filtration device 1 according to the first embodiment, as shown in Fig. 16, in a case where the entire excessive filtration pressure region generated in the first treatment cannot be included in the appropriate filtration pressure region $R_{A2}$ in the second treatment, and the entire excessive filtration pressure region generated in the second treatment cannot be included in the appropriate filtration pressure region $R_{A1}$ in the first treatment, the risk of occurrence of cell clogging increases, and the cell recovery rate may decrease in the part that becomes the excessive filtration pressure region in both the first treatment and the second treatment.

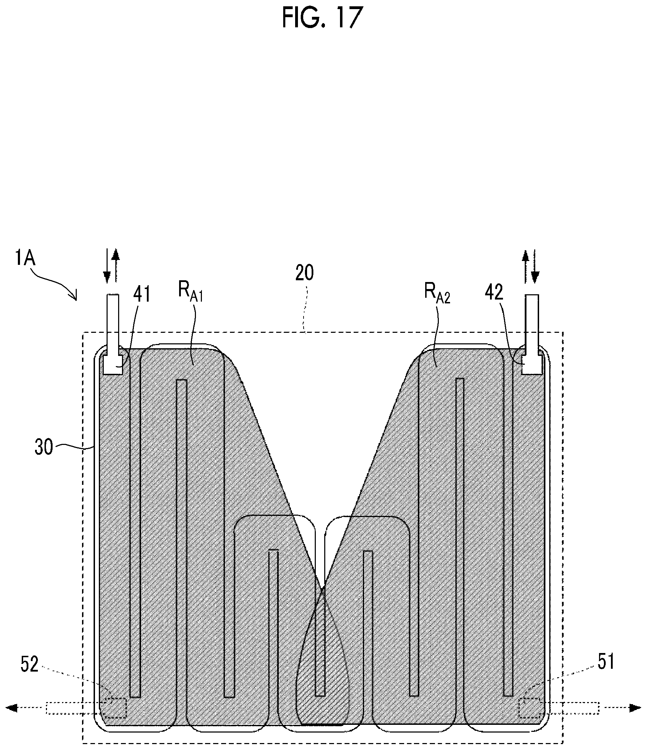

According to the filtration device 1A according to the second embodiment, as shown in Fig. 17, the flow path portion that becomes the excessive filtration pressure region is deleted in both the first treatment and the second treatment, and thereby the risk of occurrence of cell clogging can be inhibited.

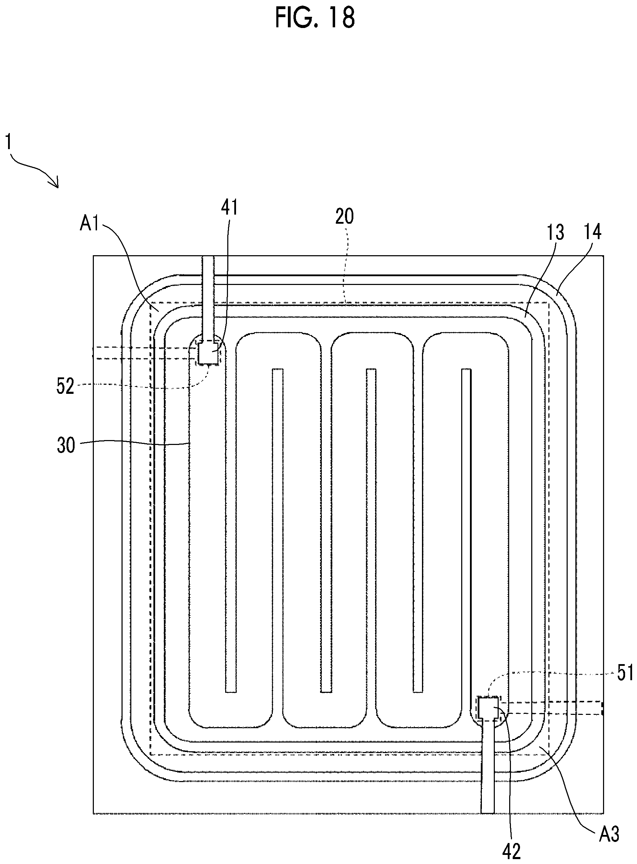

In the above description, the configuration in which the one end and the other end of the flow path 30 are disposed on the same side of the filtration membrane 20 having a rectangular shape, and accordingly, the first distribution port 41 and the second distribution port 42 are disposed at the side of the same side of the filtration membrane 20 was described, but the present invention is not limited to this aspect. For example, as shown in Fig. 18, one end of the flow path 30 and the first distribution port 41 may be disposed in the vicinity of the corner A1 of the filtration membrane 20 having a rectangular shape, and the other end of the flow path 30 and the second distribution port 42 may be disposed in the vicinity of the corner A3 that is a diagonal of the corner A1 of the filtration membrane 20. In this case, the first discharge port 51 is disposed, for example in the vicinity of corner A3 of the filtration membrane 20, that is, immediately below the second distribution port 42, and the second discharge port 52 is disposed, for example, in the vicinity of the corner A1 of the filtration membrane 20, that is, immediately below the first distribution port 41.

This application claims the priority of Japanese Patent Application No. 2017-183956, which is a Japanese application filed on September 25, 2017, the entire contents of which are incorporated herein by reference. In addition, all documents, patent applications, and technical standards described in the present specification are incorporated herein by reference to the extent in a case where it is specifically and individually stated that individual documents, patent applications, and technical standards are incorporated by reference.

**Claims**

1. A filtration device comprising:

    a flow path that meanders;
    a filtration membrane that separates a supply side and a permeation side of the flow path;
    a first distribution port that is provided at one end of the supply side of the flow path;
    a second distribution port that is provided at another end of the supply side of the flow path;
    a first discharge port that is provided on the permeation side of the flow path; and
    a second discharge port that is provided, at a position different from that of the first discharge port, on the permeation side of the flow path.

2. The filtration device according to claim 1,

    wherein, in a case in which a distance from a center of the first distribution port to a farthest position on a membrane surface of the filtration membrane is W1, and a distance from the center of the first distribution port to a center of the first discharge port is d1, and
    in a case in which a distance from a center of the second distribution port to the farthest position on the membrane surface of the filtration membrane is W2, and a distance from the center of the second distribution port to a center of the second discharge port is d2,
    $0.5W1 \leq d1 \leq W1$ is satisfied, and
    $0.5W2 \leq d2 \leq W2$ is satisfied.

3. The filtration device according to claim 1 or 2, wherein the flow path includes a plurality of folded portions, and respective distances from one of the folded portions to a next folded portion are the same as each other.

4. The filtration device according to claim 3,

wherein one end and another end of the flow path, and the respective folded portions on one side are provided on a first straight line, and the respective folded portions on another side are provided on a second straight line parallel to the first straight line.

**5.** The filtration device according to claim 1 or 2, wherein the flow path includes a plurality of folded portions, and a distance from one of the folded portions to a next folded portion is shorter than other distances.

**6.** A filtration system comprising:

the filtration device according to any one of claims 1 to 5; and a controller that performs first liquid feeding control for controlling liquid feeding of a liquid passing through the first distribution port and the second distribution port, and performs, in conjunction with the first liquid feeding control, second liquid feeding control for controlling liquid feeding of the liquid discharged from the first discharge port and the second discharge port.

**7.** The filtration system according to claim 6,

wherein the controller alternately performs, as the first liquid feeding control, first liquid feeding for discharging a liquid flowed in from the first distribution port from the second distribution port, and second liquid feeding for discharging a liquid flowed in from the second distribution port from the first distribution port, and wherein the controller performs, as the second liquid feeding control, discharge of a liquid that has passed through the filtration membrane from the first discharge port while performing the first liquid feeding, and discharge of the liquid that has passed through the filtration membrane from the second discharge port while performing the second liquid feeding.

**8.** The filtration system according to claim 6 or 7, wherein the liquid is a cell suspension.

**9.** A liquid filtration method which uses the filtration device according to any one of claims 1 to 5, the method comprising:

alternately performing first liquid feeding for allowing a liquid flowed in from the first distribution port to flow out from the second distribution port, and second liquid feeding for allowing a liquid flowed in from the second distribution port to flow out from the first distribution port; and discharging a liquid that has passed through the filtration membrane from the first discharge port while performing the first liquid feeding, and discharging the liquid that has passed through the filtration membrane from the second discharge port while performing the second liquid feeding.

**10.** The filtration method according to claim 9, wherein a cell suspension is a target of the first liquid feeding and the second liquid feeding.

# FIG. 1

## FIG. 2A

## FIG. 2B

## FIG. 2C

## FIG. 2D

# FIG. 3

## FIG. 4

START

S1 — PERMEATED LIQUID IS DISCHARGED FROM FIRST DISCHARGE PORT WHILE ALLOWING CELL SUSPENSION TO FLOW TOWARD SECOND DISTRIBUTION PORT FROM FIRST DISTRIBUTION PORT (FIRST LIQUID FEEDING)

S2 — IS THERE ABNORMALITY DURING FIRST LIQUID FEEDING?
Y
N

S3 — PERMEATED LIQUID IS DISCHARGED FROM SECOND DISCHARGE PORT WHILE ALLOWING CELL SUSPENSION TO FLOW TOWARD FIRST DISTRIBUTION PORT FROM SECOND DISTRIBUTION PORT (SECOND LIQUID FEEDING)

S4 — IS THERE ABNORMALITY DURING SECOND LIQUID FEEDING?
Y
N

S5 — THE NUMBER OF CYCLES OF TREATMENTS HAS REACHED PREDETERMINED NUMBER?
N
Y

S6 — CELL CONCENTRATION HAS REACHED TARGET VALUE?
N
Y

S7 — NOTIFICATION OF ABNORMALITY

COMPLETE

FIG. 5A

FIG. 5B

FIG. 5C

# FIG. 6A

## FIG. 6B

FILTRATION PRESSURE

POSITION ON L2

## FIG. 6C

LIQUID FEEDING SPEED

POSITION ON L2

# FIG. 7A

FIG. 7B

FIG. 7C

# FIG. 8

## FIG. 9

```
                          ( START )
                              │
S11 ─┐    ┌──────────────────────────────────────────────┐
     │    │  CONTROL VALVES V1 AND V2 TO BE IN OPEN STATE  │
          └──────────────────────────────────────────────┘
                              │
                              ▼◄──────────────────────────┐
S12 ─┐    ┌──────────────────────────────────────────────┐
     │    │            DRIVE FIRST SYRINGE PUMP           │
          │       CONTROL VALVE V4 TO BE IN OPEN STATE     │
          │      CONTROL VALVE V3 TO BE IN CLOSED STATE    │
          └──────────────────────────────────────────────┘
                              │
                              ▼
S13 ─┐            ╱ IS THERE ╲
     │      Y   ╱  ABNORMALITY IN PUMP ╲
     ◄─────────╲       LOAD?          ╱
               ╲                    ╱
                       │ N
                       ▼
S14 ─┐    ┌──────────────────────────────────────────────┐
     │    │           DRIVE SECOND SYRINGE PUMP           │
          │       CONTROL VALVE V3 TO BE IN OPEN STATE     │
          │      CONTROL VALVE V4 TO BE IN CLOSED STATE    │
          └──────────────────────────────────────────────┘
                              │
                              ▼
S15 ─┐            ╱ IS THERE ╲
     │      Y   ╱  ABNORMALITY IN PUMP ╲
     ◄─────────╲       LOAD?          ╱
                       │ N
                       ▼
S16 ─┐         ╱ THE NUMBER OF ╲
     │       ╱ CYCLES OF TREATMENTS HAS ╲   N
             ╲ REACHED PREDETERMINED    ╱ ─────►
              ╲    NUMBER?            ╱
                       │ Y
                       ▼
S17 ─┐    N    ╱ CELL ╲
     ◄────────╱ CONCENTRATION HAS REACHED ╲
              ╲     TARGET VALUE?         ╱
                       │ Y
S18 ─┐                 │
  ┌──────────────┐     │
  │ NOTIFICATION │     │
  │ OF ABNORMALITY│    │
  └──────────────┘     │
         └─────────────┤
                       ▼
                  ( COMPLETE )
```

EP 3 673 979 A1

# FIG. 10

27

# FIG. 11

# FIG. 12

FIG. 13

# FIG. 14A

COMPARATIVE EXAMPLE

# FIG. 14B

EXAMPLE

# FIG. 15

# FIG. 16

# FIG. 17

# FIG. 18

## INTERNATIONAL SEARCH REPORT

| | International application No. |
|---|---|
| | PCT/JP2018/034688 |

### A. CLASSIFICATION OF SUBJECT MATTER
Int.Cl. B01D61/18(2006.01)i, C12M3/00(2006.01)i, C12M3/06(2006.01)i

According to International Patent Classification (IPC) or to both national classification and IPC

### B. FIELDS SEARCHED

Minimum documentation searched (classification system followed by classification symbols)
Int.Cl. B01D61/18, C12M3/00, C12M3/06

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
Japio-GPG/FX

### C. DOCUMENTS CONSIDERED TO BE RELEVANT

| Category* | Citation of document, with indication, where appropriate, of the relevant passages | Relevant to claim No. |
|---|---|---|
| Y | WO 2007/102427 A1 (MANABE, Seiichi) 13 September 2007, paragraphs [0137]-[0219], fig. 1-12 & US 2009/0145831 A1, paragraphs [0212]-[0362], fig. 1-12 & EP 2006016 A1 & CN 101394917 A & AU 2007223448 A1 | 1-10 |
| Y | JP 2004-351388 A (PIGEON CORPORATION) 16 December 2004, paragraphs [0034]-[0071], fig. 1-5 (Family: none) | 1-10 |
| Y | JP 2003-024947 A (MATSUSHITA ELECTRIC INDUSTRIAL CO., LTD.) 28 January 2003, paragraphs [0014]-[0027], fig. 1-6 (Family: none) | 1-10 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 December 2018 (13.12.2018) | 25 December 2018 (25.12.2018) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/034688

C (Continuation). DOCUMENTS CONSIDERED TO BE RELEVANT

| Category* | Citation of document, with indication, where appropriate, of the relevant passages | Relevant to claim No. |
|---|---|---|
| Y | JP 62-163706 A (KURITA WATER INDUSTRIES LTD.) 20 July 1987, claims, fig. 1 (Family: none) | 1-10 |
| Y | JP 06-079142 A (MITSUBISHI HEAVY INDUSTRIES, LTD.) 22 March 1994, claim 1, fig. 1 (Family: none) | 1-10 |
| Y | JP 2004-141846 A (ORGANO CORP.) 20 May 2004, claims 1-7, fig. 1-4 & WO 2004/022206 A1 & TW 200404601 A & KR 10-2005-0033547 A | 1-10 |
| Y | JP 2002-210335 A (ORGANO CORP.) 30 July 2002, claims 1-6, fig. 1-2 (Family: none) | 1-10 |
| A | Microfilm of the specification and drawings annexed to the request of Japanese Utility Model Application No. 105427/1981 (Laid-open No. 014919/1983) (TOYO SODA MFG CO., LTD.) 29 January 1983, detailed descriptions of the invention, fig. 1-4 (Family: none) | 1-10 |
| A | JP 51-19640 Y1 (FUNAKOSHI YAKUHIN KK) 24 May 1976, detailed descriptions of the invention, fig. 1-5 (Family: none) | 1-10 |
| A | JP 02-095421 A (DAIKIN INDUSTRIES, LTD.) 06 April 1990, detailed description of the invention, fig. 1-6 (Family: none) | 1-10 |
| A | WO 2015/063490 A1 (EXMOOR PHARMA CONCEPTS LTD.) 07 May 2015, claims 1-39, fig. 1-17 & US 2016/0243502 A1 & EP 3062916 A1 | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8024590 A
- JP H08024590 A
- JP 2009291744 A
- JP 2017104832 A
- JP 2017183956 A